# EUROPEAN PATENT SPECIFICATION

(11) **EP 3 703 656 B1**
(45) Date of publication and mention of the grant of the patent: **02.08.2023**
(21) Application number: 18800274.5
(22) Date of filing: 31.10.2018
(51) Int. Cl.: A61K 9/00, A61K 9/12, A61K 9/06, A61K 31/465, A61M 15/06, A24B 15/167, A61K 47/02, A24F 40/10, A61M 11/00, A61M 11/04

(54) **AEROSOLISABLE GEL**
AEROSOLIERBARES GEL
GEL POUVANT FORMER UN AÉROSOL

(30) Priority: 01.11.2017 GB 201718032
(43) Date of publication of application: 09.09.2020
(73) Proprietor: Nicoventures Trading Limited, London WC2R 3LA (GB)
(72) Inventor: YAO, John, Cambridge Massachusetts MA 02138 (US); MCLELLAN, Joseph, Cambridge Massachusetts MA 02138 (US); LI, Xinhua, Cambridge Massachusetts MA 02138 (US); FARHAT, Hootan, Cambridge Massachusetts MA 02138 (US); BATES, Michael, Cambridge Massachusetts MA 02138 (US)
(74) Representative: Dehns
(86) International application number: PCT/GB2018/053140
(87) International publication number: WO 2019/086860

(56) References cited:
- EP-A1- 1 151 751
- EP-A2- 0 405 190
- WO-A1-2012/019372
- WO-A2-2013/036415
- CN-A- 101 347 477
- CN-A- 101 979 011
- CN-A- 102 125 028
- DE-A1- 19 854 009
- JP-A- 2007 308 443
- US-A1- 2015 209 530
- US-A1- 2017 266 087
- DATABASE WPI Week 197844 Thomson Scientific, London, GB; AN 1978-78926A XP002113399, -& JP S53 109933 A (SAKAI SHOJI KK) 26 September 1978 (1978-09-26)

## Description

### FIELD OF THE INVENTION

The present disclosure relates to a dosage form of an aerosolisable gel product, a method of forming an aerosol and to electronic vapour provision systems such as electronic delivery systems (e.g. e-cigarettes) incorporating said aerosolisable gel.

### BACKGROUND TO THE INVENTION

Electronic vapour provision systems such as e-cigarettes generally contain a reservoir of liquid which is to be vaporised, typically containing nicotine. When a user inhales on the device, a heater is activated to vaporise a small amount of liquid, which is therefore inhaled by the user.

The use of e-cigarettes in the UK has grown rapidly, and it has been estimated that there are now over a million people using them in the UK.

Current electronic cigarettes generate an aerosol by vaporizing a consumable e-liquid composed mostly of propylene glycol (PG), glycerol, water, nicotine and flavours. The e-liquid is drawn by a wicking material into a resistive heating coil in which it is heated and evaporated. This straight-forward system suffers from a number of drawbacks such as leaking of the e-liquid though gaskets and the mouthpiece, inefficient heating and an inconsistent aerosol composition.

EP 0405190 relates to a cigarette which heats but does not burn tobacco. The cigarette comprises a carbonaceous fuel element extruded around a separate aerosol generating means comprising an aerosol former material and inductive or electric heating means. The aerosol generating means of EP 0405190 comprises an admixture of bentonite clay and glycerine in water.

DE 19854009 relates to a system for supplying an inhalable aerosol. The nebuliser comprises nicotine, polyols, water and a material-carrier, such as sepiolite.

CN 102125028 discloses a paste for preventing trunk decaying pests comprising pesticide such as nicotine, koaolin, glycerol, thickening agent and water.

US 2017/266087 relates to a cosmetic composition containing cosmetically active agents. The composition comprises synthetic phyllosilicate gel, water, glycerol and active agents.

JP S53109933 relates to the treatment of a fever. The disclosed compositions include active ingredients such as menthol absorbed onto materials such as kaolin.

JP 2007308443 relates to a tooth whitening composition for use in the oral cavity. The composition comprises ascorbic acid ester, gelling agent such as sodium carboxymethyl cellulose or carrageenan, kaolin, glycerol or propylene glycol and water.

CN 101979011 discloses a medicinal product with good transdermal delivery for the treatment of cervical spondylosis. The composition comprises an active agent, gelling agents such as sodium carboxymethyl cellulose, gelatin or polyacrylate, kaolin, sorbic acid, menthol, terpenes, geraniol, glycerine, propylene glycol or their mixture and water.

CN 101347477 relates to a pharmaceutical composition for the treatment of rheumatoid arthritis. The composition comprises gelatin, sodium polyacrylate, kaolin, glycerol, zinc oxide and water.

EP 1151751 relates to gel compositions formed by a crosslinked polymer gel system. The compositions comprise vitamin C, kaolin, glycerol and water.

US 2015/209530 describes substances for use with an aerosol agent delivery device to vaporise and deliver an aerosol agent to a user. The composition comprises a binder material, an aerosol agent, such as a therapeutic drug or nicotine, an aerosol forming agent and an inorganic, inert filler material.

WO 2013/036415 describes the use of nano-sized clay minerals to enhance the viscosity of aqueous fluids that have increased viscosity due to the presence of viscoelastic surfactants (VESs).

WO 2012/019372 describes a medicinal healthcare solid electronic cigarette atomising solution comprising propylene glycol, medicament, water, tobacco leaf extract solution, cigarette essence, excipient, calcium pectate gelatin and curing agent.

### SUMMARY OF THE INVENTION

In one aspect there is provided a dosage form of an aerosolisable gel product, wherein the dosage form of the aerosolisable gel comprises
(a) nicotine in an amount of 0.5 to 10 mg;
(b) one or more gel forming materials, wherein the one or more gel forming materials is at least kaolinite;
(c) an aerosol forming material; and
(d) water.

In one aspect there is provided a method of forming an aerosol, the method comprising the step of heating an aerosolisable gel comprising
(a) an active agent;
(b) a gel forming material, wherein the gel forming material is at least kaolinite;
(c) an aerosol forming material; and
(d) water.

In one aspect there is provided an electronic vapour provision system comprising:
(i) an aerosolisable gel comprising
   (a) an active agent;
   (b) one or more gel forming materials, wherein the one or more gel forming material is at least kaolinite;
   (c) an aerosol forming material; and
   (d) water;
(ii) a heater for heating the aerosolisable gel and thereby vaporising some or all of the aerosolisable gel for inhalation by a user of the electronic vapour provision system.

### DETAILED DESCRIPTION

As discussed herein the present invention provides a dosage form of an aerosolisable gel product comprising
(a) nicotine in an amount of 0.5 to 10 mg;
(b) one or more gel forming materials, wherein the one or more gel forming material is at least kaolinite; (c) an aerosol forming material; and (d) water.

We have found that we may avoid many of the limitations of the e-liquid of the prior art by using a non-liquid gel-medium. Gel-media will allow greater flexibility in the design of future heating technologies and consumable form factors. We have found that the gel heats and vaporizes efficiently. We have also found that the gel consistently delivers the active agent, such as nicotine, and flavours in the aerosol. In particular, the resulting aerosol does not contain any detectable toxicants or malodour component resulting from the thermal degradation of the gel.

We have identified that via selection of a particular gel forming material, namely kaolinite, the problems of the prior art my may be overcome. In particular, we have found that the gel materials containing kaolinite exhibit a similar heating profile compared to 'raw' liquid. In contrast many prior art gels require significantly longer heating times to aerosolise the same amount of liquid, indicating significantly inhibited heat transfer in these samples. Furthermore, in contrast to prior art samples, the aerosolisable gel of the present invention containing kaolinite achieved close to 100% aerosolisation of the liquid contained therein. Yet further, the inorganic gelling material used in the present invention, namely kaolinite provides a thermally stable gelling material and therefore avoids the possibility of toxicants or malodorous components being provided to the user through the thermal degradation of the gel.

For ease of reference, these and further aspects of the present invention are now discussed under appropriate section headings. However, the teachings under each section are not necessarily limited to each particular section.

### Active agent

The aerosolisable gel comprises an active agent. By "active agent" it is meant an agent which has a biological effect on a subject when the aerosol is inhaled. The one or more active agents may be selected from nicotine, botanicals, and mixtures thereof. The one or more active agents may be of synthetic or natural origin. The active could be an extract from a botanical, such as from a plant in the tobacco family. An example active is nicotine.

In one aspect, the active agent is at least nicotine. Nicotine may be provided at any suitable amount depending on the desired dosage when inhaled by the user. In one aspect nicotine is present in an amount of no greater than 6 wt% based on the total weight of the aerosolisable gel. In one aspect nicotine is present in an amount of from 0.4 to 6 wt% based on the total weight of the aerosolisable gel. In one aspect nicotine is present in an amount of from 0.8 to 6 wt% based on the total weight of the aerosolisable gel. In one aspect nicotine is present in an amount of from 1 to 6 wt% based on the total weight of the aerosolisable gel. In one aspect nicotine is present in an amount of from 1.8 to 6 wt% based on the total weight of the aerosolisable gel. In one aspect nicotine is present in an amount of from 0.4 to 5 wt% based on the total weight of the aerosolisable gel. In one aspect nicotine is present in an amount of from 0.8 to 5 wt% based on the total weight of the aerosolisable gel. In one aspect nicotine is present in an amount of from 1 to 5 wt% based on the total weight of the aerosolisable gel. In one aspect nicotine is present in an amount of from 1.8 to 5 wt% based on the total weight of the aerosolisable gel. In one aspect nicotine is present in an amount of no greater than 4 wt% based on the total weight of the aerosolisable gel. In one aspect nicotine is present in an amount of from 0.4 to 4 wt% based on the total weight of the aerosolisable gel. In one aspect nicotine is present in an amount of from 0.8 to 4 wt% based on the total weight of the aerosolisable gel. In one aspect nicotine is present in an amount of from 1 to 4 wt% based on the total weight of the aerosolisable gel. In one aspect nicotine is present in an amount of from 1.8 to 4 wt% based on the total weight of the aerosolisable gel. In one aspect nicotine is present in an amount of no greater than 3 wt% based on the total weight of the aerosolisable gel. In one aspect nicotine is present in an amount of from 0.4 to 3 wt% based on the total weight of the aerosolisable gel. In one aspect nicotine is present in an amount of from 0.8 to 3 wt% based on the total weight of the aerosolisable gel. In one aspect nicotine is present in an amount of from 1 to 3 wt% based on the total weight of the aerosolisable gel. In one aspect nicotine is present in an amount of from 1.8 to 3 wt% based on the total weight of the aerosolisable gel. In one aspect nicotine is present in an amount of no greater than 1.9 wt% based on the total weight of the aerosolisable gel. In one aspect nicotine is present in an amount of no greater than 1.8 wt% based on the total weight of the aerosolisable gel. In one aspect nicotine is present in an amount of from 0.4 to 1.9 wt% based on the total weight of the aerosolisable gel. In one aspect nicotine is present in an amount of from 0.4 to 1.8 wt% based on the total weight of the aerosolisable gel. In one aspect nicotine is present in an amount of from 0.5 to 1.9 wt% based on the total weight of the aerosolisable gel. In one aspect nicotine is present in an amount of from 0.5 to 1.8 wt% based on the total weight of the aerosolisable gel In one aspect nicotine is present in an amount of from 0.8 to 1.9 wt% based on the total weight of the aerosolisable gel.

In one aspect nicotine is present in an amount of from 0.8 to 1.8 wt% based on the total weight of the aerosolisable gel. In one aspect nicotine is present in an amount of from 1 to 1.9 wt% based on the total weight of the aerosolisable gel. In one aspect nicotine is present in an amount of from 1 to 1.8 wt% based on the total weight of the aerosolisable gel. In one aspect nicotine is present in an amount of less than 1.9 wt% based on the total weight of the aerosolisable gel. In one aspect nicotine is present in an amount of less than 1.8 wt% based on the total weight of the aerosolisable gel. In one aspect nicotine is present in an amount of from 0.4 to less than 1.9 wt% based on the total weight of the aerosolisable gel. In one aspect nicotine is present in an amount of from 0.4 to less than 1.8 wt% based on the total weight of the aerosolisable gel. In one aspect nicotine is present in an amount of from 0.5 to less than 1.9 wt% based on the total weight of the aerosolisable gel. In one aspect nicotine is present in an amount of from 0.5 to less than 1.8 wt% based on the total weight of the aerosolisable gel. In one aspect nicotine is present in an amount of from 0.8 to less than 1.9 wt% based on the total weight of the aerosolisable gel. In one aspect nicotine is present in an amount of from 0.8 to less than 1.8 wt% based on the total weight of the aerosolisable gel. In one aspect nicotine is present in an amount of from 1 to less than 1.9 wt% based on the total weight of the aerosolisable gel. In one aspect nicotine is present in an amount of from 1 to less than 1.8 wt% based on the total weight of the aerosolisable gel. In one aspect nicotine is present in an amount of no greater than 2 wt% based on the total weight of the aerosolisable gel. In one aspect nicotine is present in an amount of no greater than 1.8 wt% based on the total weight of the aerosolisable gel.

In the context of the present invention, reference to nicotine includes nicotine in both protonated form and in unprotonated form.

In one aspect the gel comprises nicotine in unprotonated form and nicotine in protonated form. In one aspect the gel comprises nicotine in unprotonated form and nicotine in monoprotonated form. Although it is envisaged that the gel typically comprise nicotine in unprotonated form and nicotine in monoprotonated form, it may be that small amounts of diprotonated nicotine are present. In one aspect the gel comprises nicotine in unprotonated form, nicotine in monoprotonated form and nicotine in diprotonated form. As will be understood by one skilled in the art, the protonated form of nicotine is prepared by reacting unprotonated nicotine with an acid. The acids are one or more suitable acids. In one aspect the acid is an organic acid. In one aspect the acid is a carboxylic acid. The carboxylic acid may be any suitable carboxylic acid. In one aspect the acid is a mono-carboxylic acid. In one aspect the acid is selected from the group consisting of benzoic acid, levulinic acid, acetic acid, lactic acid, formic acid, citric acid, pyruvic acid, succinic acid, tartaric acid, oleic acid, sorbic acid, propionic acid, phenylacetic acid, and mixtures thereof.

### Gel Forming Materials

As discussed herein the present invention provides an aerosolisable gel comprising one or more gel forming materials, wherein the one or more gel forming material is at least kaolinite. As will be understood by one skilled in the art, kaolinite is a clay mineral, part of the group of industrial minerals, with the chemical composition Al₂Si₂O₅(OH)₄. It is a layered silicate mineral, with one tetrahedral sheet of silica (SiO₄) linked through oxygen atoms to one octahedral sheet of alumina (AlO₆) octahedra. Rocks that are rich in kaolinite are known as kaolin or china clay. References herein to kaolin are to be read as references to kaolinite.

The gel forming material and kaolinite in particular may be provided at any suitable amount depending on the desired composition of the final gel. In one aspect kaolinite is present in an amount of 10 to 90 wt.% based on the total weight of the aerosolisable gel. In one aspect kaolinite is present in an amount of 10 to 80 wt.% based on the total weight of the aerosolisable gel. In one aspect kaolinite is present in an amount of 20 to 80 wt.% based on the total weight of the aerosolisable gel. In one aspect kaolinite is present in an amount of 30 to 80 wt.% based on the total weight of the aerosolisable gel. In one aspect kaolinite is present in an amount of 20 to 70 wt.% based on the total weight of the aerosolisable gel. In one aspect kaolinite is present in an amount of 30 to 70 wt.% based on the total weight of the aerosolisable gel. In one aspect kaolinite is present in an amount of 20 to 60 wt.% based on the total weight of the aerosolisable gel. In one aspect kaolinite is present in an amount of 30 to 60 wt.% based on the total weight of the aerosolisable gel. In one aspect kaolinite is present in an amount of 40 to 60 wt.% based on the total weight of the aerosolisable gel.

As will be appreciated by one skilled in the art, in one aspect the aerosolisable gel may contain other gel forming materials in addition to kaolinite. In one aspect the aerosolisable gel contains no other gel forming materials in addition to kaolinite. Thus in one aspect the aerosolisable gel comprises gel forming materials consisting of kaolinite.

In one aspect the one or more gel forming materials is present in an amount of 10 to 90 wt.% based on the total weight of the aerosolisable gel. In one aspect the one or more gel forming materials is present in an amount of 10 to 80 wt.% based on the total weight of the aerosolisable gel. In one aspect the one or more gel forming materials is present in an amount of 20 to 80 wt.% based on the total weight of the aerosolisable gel. In one aspect the one or more gel forming materials is present in an amount of 30 to 80 wt.% based on the total weight of the aerosolisable gel. In one aspect the one or more gel forming materials is present in an amount of 20 to 70 wt.% based on the total weight of the aerosolisable gel. In one aspect the one or more gel forming materials is present in an amount of 30 to 70 wt.% based on the total weight of the aerosolisable gel. In one aspect the one or more gel forming materials is present in an amount of 20 to 60 wt.% based on the total weight of the aerosolisable gel. In one aspect the one or more gel forming materials is present in an amount of 30 to 60 wt.% based on the total weight of the aerosolisable gel. In one aspect the one or more gel forming materials is present in an amount of 40 to 60 wt.% based on the total weight of the aerosolisable gel.

### Aerosol Forming Material

The aerosol forming material of the aerosolisable gel is provided such that the aerosolisable gel produces a vapour under the appropriate heating conditions. The aerosol forming material may contain aerosol forming components such as glycerol, propylene glycol and mixtures thereof. In one aspect the aerosol forming material is at least glycerol. In one aspect the aerosol forming material consists essentially of glycerol. In one aspect the aerosol forming material consists of glycerol. In one aspect the aerosol forming material is at least propylene glycol. In one aspect the aerosol forming material consists essentially of propylene glycol. In one aspect the aerosol forming material consists of propylene glycol. In one aspect the aerosol forming material is at least a mixture of propylene glycol and glycerol. In one aspect the aerosol forming material consists essentially of a mixture of propylene glycol and glycerol. In one aspect the aerosol forming material consists of a mixture of propylene glycol and glycerol.

The aerosol forming material of the aerosolisable gel may be present in any suitable amount. In one aspect the aerosol forming material is present in an amount of 1 to 90 wt% based on the aerosolisable gel. In one aspect the aerosol forming material is present in an amount of 5 to 90 wt% based on the aerosolisable gel. In one aspect the aerosol forming material is present in an amount of 10 to 90 wt% based on the aerosolisable gel. In one aspect the aerosol forming material is present in an amount of 10 to 80 wt% based on the aerosolisable gel. In one aspect the aerosol forming material is present in an amount of 10 to 70 wt% based on the aerosolisable gel. In one aspect the aerosol forming material is present in an amount of 10 to 60 wt% based on the aerosolisable gel. In one aspect the aerosol forming material is present in an amount of 20 to 60 wt% based on the aerosolisable gel. In one aspect the aerosol forming material is present in an amount of 10 to 50 wt% based on the aerosolisable gel. In one aspect the aerosol forming material is present in an amount of 20 to 50 wt% based on the aerosolisable gel. In one aspect the aerosol forming material is present in an amount of 10 to 40 wt% based on the aerosolisable gel. In one aspect the aerosol forming material is present in an amount of 20 to 40 wt% based on the aerosolisable gel.

### Water

The aerosolisable gel further comprises water. The water may be present in any suitable amount. In one aspect water is present in an amount of 1 to 50 wt% based on the aerosolisable gel. In one aspect water is present in an amount of 5 to 50 wt% based on the aerosolisable gel. In one aspect water is present in an amount of 1 to 45 wt% based on the aerosolisable gel. In one aspect water is present in an amount of 5 to 45 wt% based on the aerosolisable gel. In one aspect water is present in an amount of 1 to 40 wt% based on the aerosolisable gel. In one aspect water is present in an amount of 5 to 40 wt% based on the aerosolisable gel. In one aspect water is present in an amount of 1 to 35 wt% based on the aerosolisable gel. In one aspect water is present in an amount of 5 to 35 wt% based on the aerosolisable gel. In one aspect water is present in an amount of 1 to 30 wt% based on the aerosolisable gel. In one aspect water is present in an amount of 5 to 30 wt% based on the aerosolisable gel. In one aspect water is present in an amount of 1 to 25 wt% based on the aerosolisable gel. In one aspect water is present in an amount of 5 to 25 wt% based on the aerosolisable gel. In one aspect water is present in an amount of 1 to 20 wt% based on the aerosolisable gel. In one aspect water is present in an amount of 5 to 20 wt% based on the aerosolisable gel. In one aspect water is present in an amount of 1 to 15 wt% based on the aerosolisable gel. In one aspect water is present in an amount of 5 to 15 wt% based on the aerosolisable gel.

In one aspect the combined amount of aerosol forming material and water in the aerosolisable gel is from 1 to 90 wt% based on the aerosolisable gel. In one aspect the combined amount of aerosol forming material and water in the aerosolisable gel is 5 to 90 wt% based on the aerosolisable gel. In one aspect the combined amount of aerosol forming material and water in the aerosolisable gel is 1 to 80 wt% based on the aerosolisable gel. In one aspect the combined amount of aerosol forming material and water in the aerosolisable gel is 5 to 80 wt% based on the aerosolisable gel. In one aspect the combined amount of aerosol forming material and water in the aerosolisable gel is 10 to 80 wt% based on the aerosolisable gel. In one aspect the combined amount of aerosol forming material and water in the aerosolisable gel is 10 to 70 wt% based on the aerosolisable gel. In one aspect the combined amount of aerosol forming material and water in the aerosolisable gel is 15 to 70 wt% based on the aerosolisable gel. In one aspect the combined amount of aerosol forming material and water in the aerosolisable gel is 10 to 60 wt% based on the aerosolisable gel. In one aspect the combined amount of aerosol forming material and water in the aerosolisable gel is 15 to 60 wt% based on the aerosolisable gel. In one aspect the combined amount of aerosol forming material and water in the aerosolisable gel is 20 to 60 wt% based on the aerosolisable gel. In one aspect the combined amount of aerosol forming material and water in the aerosolisable gel is 25 to 60 wt% based on the aerosolisable gel. In one aspect the combined amount of aerosol forming material and water in the aerosolisable gel is 20 to 50 wt% based on the aerosolisable gel. In one aspect the combined amount of aerosol forming material and water in the aerosolisable gel is 25 to 50 wt% based on the aerosolisable gel. In one aspect the combined amount of aerosol forming material and water in the aerosolisable gel is 30 to 50 wt% based on the aerosolisable gel.

### Further Components

The gel may also comprise flavouring components. As used herein, the terms "flavour" and "flavourant" refer to materials which, where local regulations permit, may be used to create a desired taste or aroma in a product for adult consumers. Thus, whilst it is acknowledged that some other functional components of the formulation may contain components that have a perceptible flavour or aroma, such components are not added for this purpose and as such are not considered to be a "flavour" or "flavourant" in the context of the present invention. Furthermore, it will be understood that "flavours" or "flavourants" may well composed of one or more individual compounds that together form an identifiable flavour. As such, reference here to "flavour" or "flavourant" includes both singular and multi-component flavours. They may include extracts (e.g. liquorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g. sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

### Dosage Form

As discussed herein in one aspect, the present invention provides a dosage form of an aerosolisable gel product, wherein the dosage form of the aerosolisable gel comprises
(a) nicotine in an amount of 0.5 to 10 mg;
(b) one or more gel forming materials, wherein the one or more gel forming materials is at least kaolinite;
(c) an aerosol forming material; and
(d) water.

In one aspect the nicotine is present in an amount of 0.5 to 5 mg. In one aspect the nicotine is present in an amount of 0.5 to 4 mg.

In one aspect the nicotine is present in an amount of 0.5 to 3 mg. In one aspect the nicotine is present in an amount of 0.5 to 2 mg. In one aspect the nicotine is present in an amount of 1 to 5 mg.

In one aspect the nicotine is present in an amount of 1 to 4 mg. In one aspect the nicotine is present in an amount of 1 to 3 mg. In one aspect the nicotine is present in an amount of 1 to 2 mg.

### Method

As discussed herein in one aspect, the present invention provides a method of forming an aerosol, the method comprising the step of heating an aerosolisable gel comprising
(a) an active agent;
(b) a gel forming material, wherein the gel forming material is at least kaolinite;
(c) an aerosol forming material; and
(d) water.

### Electronic Vapour Provision System

The aerosolisable gel may be contained or delivered by any means.

Throughout the following description the term "e-cigarette" is used; however, this term may be used interchangeably with electronic vapour provision system. As discussed herein, the aerosolisable gel of the present invention is typically provided for the delivery of formulation to or within an e-cigarette. The aerosolisable gel may be held within an e-cigarette or may be sold separately, for example as one unit doses, for subsequent use with or in an e-cigarette. In one aspect the aerosolisable gel is contained within an e-cigarette. Therefore in a further aspect the present invention provides an electronic vapour provision system comprising: (i) an aerosolisable gel comprising (a) an active agent; (b) one or more gel forming materials, wherein the one or more gel forming material is at least kaolinite; (c) an aerosol forming material; and (d) water; (ii) a heater for heating the aerosolisable gel and thereby vaporising some or all of the aerosolisable gel for inhalation by a user of the electronic vapour provision system.

### BRIEF DESCRIPTION OF THE DRAWINGS

The present invention will now be described in further detail by way of example only with reference to the accompanying figure in which:
Figure 1: Gels are composed of gel forming scaffolds holding onto a base e-liquid.
Figure 2a: SEM micrograph of kaolin clay (Barrisurf XD)
Figure 2b: Gels made with kaolin clay loading levels between 30% and 60%
Figure 3: Calculated energy of vaporization for the e-liquid and 55% kaolin gel. The total energy includes heat capacity contributions from each element of the gel as well as latent heat of evaporation contributions for the e-liquid components.
Figure 4a: Evaporation rate data for various gels. Initial mass loss vs. time collected on a 10 puff equivalent samples on a 250°C hot plate
Figure 4b: Zoom of shaded are from Figure 4a (first 200 seconds)
Figure 4c: Time required to evaporate 99% of the e-liquid for various gels. Data collected using the rapid heating apparatus
Figure 4d: Current-voltage energy consumed while heating samples from Figure 4a.
Figure 5: Aerosol generation/collection apparatus.
Figure 6: Aerosol composition.
Figure 7: Thermogravimetric analysis data for kaolinite gel product.

The invention will now be described with reference to the following example.

### Examples

### Formulations

Base e-liquid:
   Propylene glycol 25 wt.%
   Water 25 wt.%
   Nicotine 1.86 wt.%
   Glycerol 48.14 wt.%
Kaolinite - Barrisurf XD available from Imerys
For 10 g batch of 60% Kaolinite gel, mix 6 g Kaolinite with 4 g e-liquid

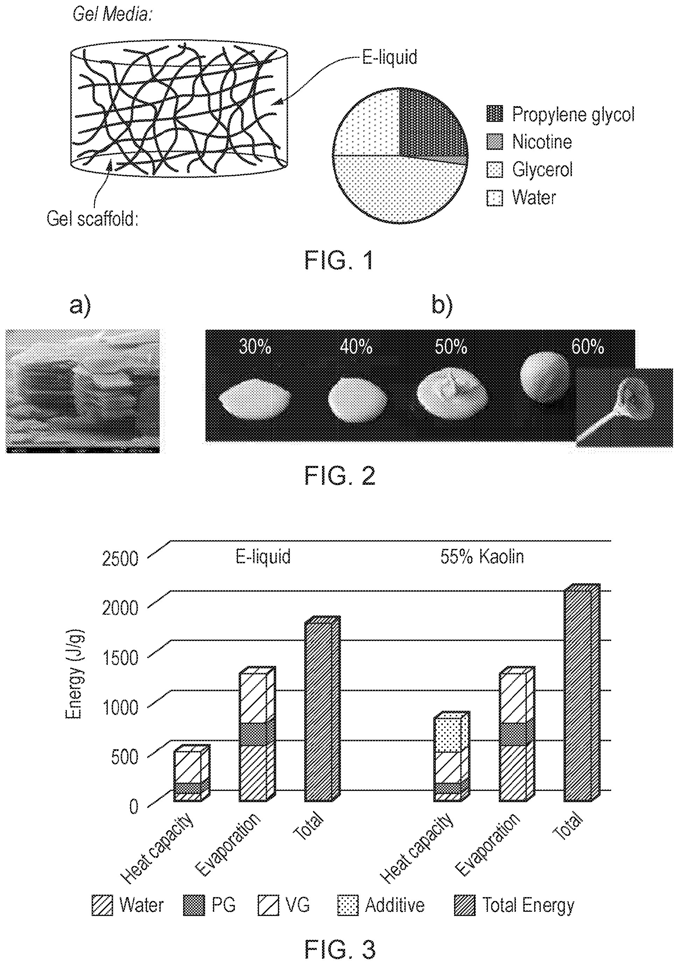

The formulations described herein provided a gelled product comprising propylene glycol, nicotine, glycerol and water held together with a gel scaffold in the manner shown in Figure 1.

Kaolin clay is an aluminosilicate (Al₂Si₂O₅(OH)₄) consisting of stacked platelets (Figure 2a). We used Barrisurf HX, a kaolin supplied as a powder from Imerys with a shape factor of -100. The kaolin powder was combined as-supplied with the e-liquid and manually stirred until the mixture was homogeneous. Figure 2b shows the consistency of the mixtures with kaolin loading levels from 30% to 60%. Over this range, the mixture takes on a consistency ranging from thick liquid to a modelling clay-like putty. Like many clay-based pastes, these gels have a shear thinning rheology.

### Testing results:

### Energy of Vaporization

For formulations with significant loading levels of additives, the added thermal mass of the additives should be considered. A crude estimate of the energy required to vaporize each formulation can be made by adding up the energy required to heat each component to its boiling point and the latent heat for each component to be vaporized. The contribution from each component is shown in Figure 3 for the e-liquid as well as kaolin gels Despite only comprising 25% of the formulation, the water accounts for a significant portion of the energy require to vaporize the e-liquid.

Table: Energy required to evaporate various gel from heat flow data obtained using differential scanning calorimetry. The total energy was calculated by integrated the heat flow data from 25°C to 300°C.

| | **△H (kJ/g)** |
|---|---|
| **E-liquid** | 1.39 |
| **55% Kaolin** | 1.90 |

Experimentally the energy required to vaporize the gels were measured by differential scanning calorimetry. The above Table shows the integrated heat flow in the heating range from 25°C to 300°C. This measurement confirms that the added contribution to the energy required to vaporize from significant levels of kaolinite is minor.

### Evaporation Kinetics:

The evaporation rate of the gels was determined gravimetrically by measuring the mass loss as a function of heating time. Initial measurements were performed by heating gel samples on hot plate - later, this was replaced by faster heating aerosol test rig. Sample sizes were chosen such that samples from the different formulations each contain the same mass of e-liquid (5-10 puff equivalents).

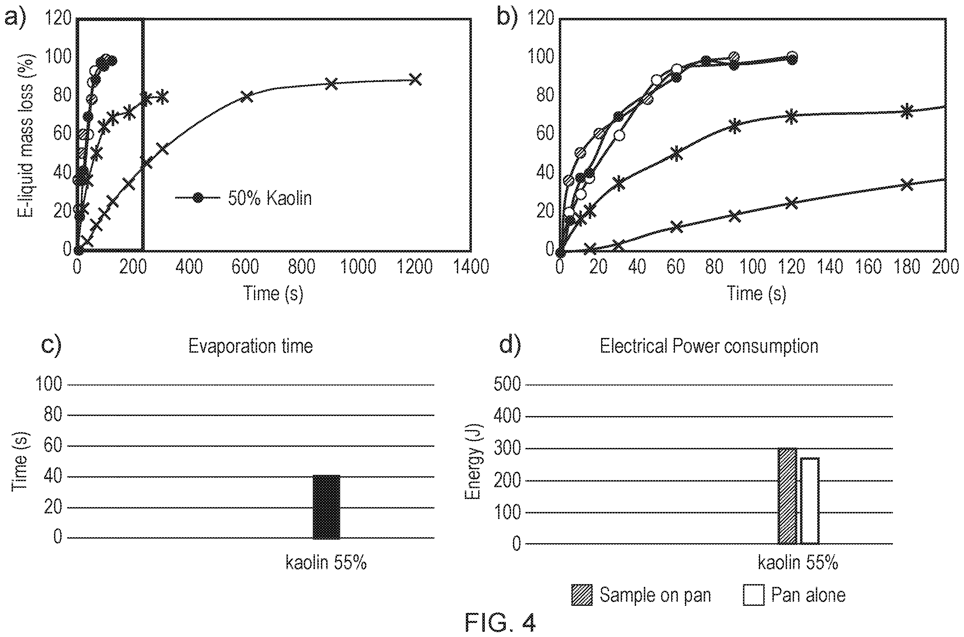

Figure 4a shows the mass loss vs time for kaolinite gel. These measurements were performed on a 10 puff equivalent (containing ~1 mg nicotine) and were heated on a 250°C hotplate. On this time-scale, the kaolinite gel exhibited similar heating profiles compared to the raw e-liquid.

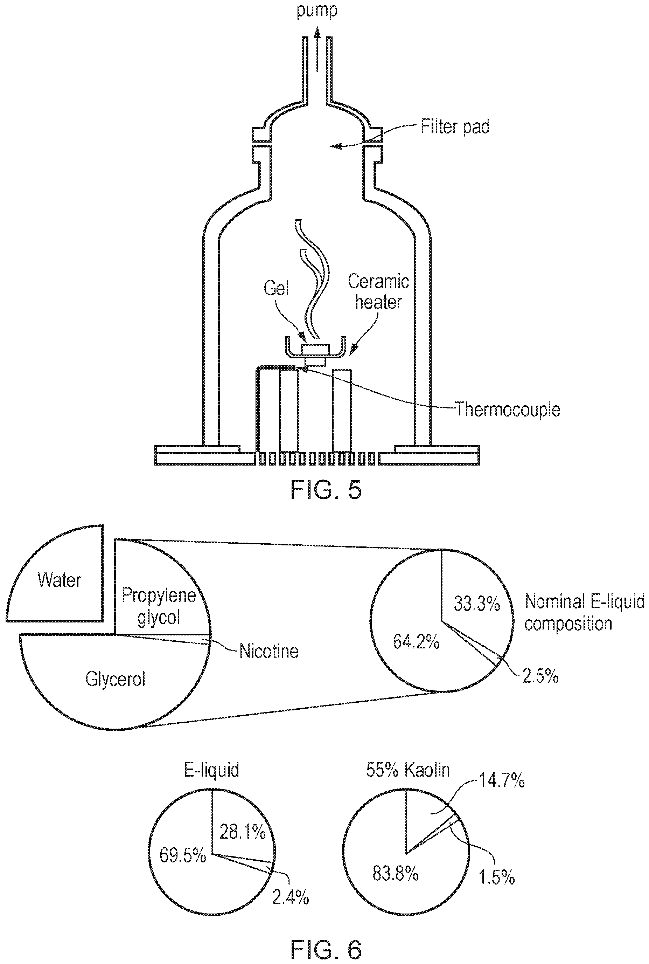

Aerosol test rig: In order to heat the gels on a time-scale closer to that of an e-cigarette device, we constructed a test rig for rapidly heating the gel and collecting the aerosol (Figure 5). All subsequent evaporation rate measurements were performed on this device. The heating element (a ceramic disc resistive element) was used to heat samples in a 25 mg aluminium pan. A set-point of 300°C can be reached within ~3-5 seconds. The heater sits in a glass chamber which is used to collect the aerosol onto a glass filter.

Figure 4c shows the heating time and energy required to aerosolize 55% kaolinite gel. The sample (5 puff equivalent) was heated to 300 °C and held for set durations. The change in sample mass was measured to determine the time required to vaporize 99% of the e-liquid in the sample. In addition to this gravimetric analysis, we also monitored power consumption to vaporize the sample and compared it to the energy required to heat the aluminium pan alone for that duration (Figure 4d).

### Aerosol Extraction and GC/MS Analysis:

The composition of the aerosols generated from the five puff gel equivalents were analysed by gas chromatography-mass spectrometry (GC/MS). The aerosol generated on the heating apparatus was collected onto a glass filter pad by evacuating the chamber with a vacuum pump. Deposits on the filter pad and on the chamber walls were extracted in methanol and analysed.

The aerosol was collected by placing an inverted Sterlitech glass filtration apparatus (Cat. # 311420) over the heater, with a Whatman 47 mm glass microfiber filter pad (Cat. # 1821-047) attached to the apparatus to collect the aerosol. The filtration apparatus was connected to an Airpo pump (model # D2028B), with a maximum pump-rate of 12-15 LPM. The pump was set to begin running upon initiation of heating and to continue running for at least 10 seconds after the sample e-liquid was completely aerosolized. The filter was then placed in 10 mL of anhydrous methanol (Sigma-Aldrich Cat. # 322415) in a 100 mL glass jar. The aerosol was extracted from the filter pad by gentle shaking of the jar for 30-60 sec. After initial extraction, the filter pad was removed from the methanol extraction solvent, squeezed to remove excess methanol and used to wipe the area around the sample holder pan and the exposed surfaces of the heating rig. The filter was then placed back in the methanol solvent to extract for another 30-60 sec, then removed from methanol, squeezed dry and used to wipe any remaining aerosol residue from the inside of the glass filtration apparatus. The filter pad was then placed back into the methanol solvent for a final 1-2 min of extraction, after which it was removed from the solvent, squeezed dry one final time and discarded. The methanol with the extracted aerosol was transferred into a 1 mL GC/MS vial with a rubber-septum cap and analysed using an auto-liquid sampler attached to the Agilent 7820A GC with 5977E MSD. The GC/MS procedure used a DB-WAX GC column (30 m length, 0.25 mm diameter, 0.25 µm film thickness, Cat. # 122-7032) for analyte separation. The procedure was optimized to resolve the peaks corresponding to: propylene glycol, nicotine and glycerol. The GC procedure is as follows:

| **Parameter** | **Value** |
|---|---|
| **Oven:** | |
| Initial Temp. | 50 °G |
| Rate | 30 °C/min |
| Final Temp. | 260 °C |
| Final Hold Time | 2 min |

| **Inlet:** | |
|---|---|
| Temp. | 300 °C |
| Mode | Split |
| Split Ratio | 10:1 |
| Split Flow Rate | 20 mL/min |
| Pressure | 16.9 psi |

| **Column:** | |
|---|---|
| Model # | 122-7032 |
| Description | DB-WAX |
| Max Temp. | 260 °C |
| Length | 30 m |
| Diameter | 0.25 mm |
| Film Thickness | 0.25 um |
| Mode | Constant Flow |
| Flow Rate | 2 mL/min |
| Average Velocity | 51.5 cm/sec |

To quantify the relative ratios of the e-liquid components, the peak areas from aerosol extraction results were compared to peak areas of a set of standards. The standards used to produce a calibration curve were made by diluting pure e-liquid in anhydrous methanol. The e-liquid was first diluted 100-fold by pipetting 100 uL of e-liquid into 9.9 mL of anhydrous methanol using micropipettes. The standards were then further diluted and labelled according to their nicotine content. The stock 1:100 dilution contained 1860 ppm nicotine and was used to make standards containing 10, 20, 30, 40 & 50 ppm nicotine. The anhydrous methanol was run as a blank, followed by the calibration curve standards. A fresh set of standards was made and analysed for each series of extraction samples to ensure accurate calibration.

We found it challenging to quantify the water content of the aerosol due to varying amounts absorbed during the aerosol collection. The water absorption could not be accounted for by running a blank filter through the extraction process. We, therefore, focused on quantifying the relative compositions of the remaining three components of the aerosol. The results are presented in the form of pie-charts in Figure 6. The composition of the e-liquid was very close to the nominal e-liquid composition. The 55%-Kaolinite gel contains an increased proportion of glycerol. Because these samples required a longer heating time therefore also a longer pumping time, we suspect that the PG and nicotine components may be pulled through the collection pad along with some water content.

### Thermal Stability of Additives:

Although the only volatile components of the formulated gels are the e-liquid components, compounds produced by thermal degradation of the gels could contribute volatile toxicants and undesirable flavour to the aerosol. Thermogravimetric analysis was performed on the gel additives to characterize the temperature at which they decomposed and give-off volatile components.

Thermogravimetric analysis on the dry powder gel additives was performed using a Shimadzu TGA-50. Each sample weighed 15-20 mg and were heated in an open aluminium pan (VWR 12577-060). The additives were heated to 600°C at a rate of 20 °C/min in an air atmosphere at a flow rate of 100 mL/min. Combined thermogravimetric analysis and differential scanning calorimetry were performed of fully formulated gels using a TA Instruments Q600. The samples were placed in covered alumina pans and an identical covered alumina pan was used for the DSC reference. Simultaneous TGA and DSC data was collected analysed by heating to 400 °C at 10 °C/min with a gas flow rate of 100 mL/min air.

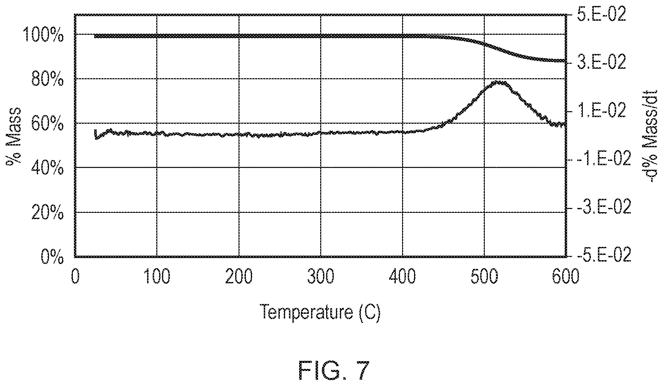

Kaolinite did not show any signs of degradation in the relevant temperature range. Thermogravimetric analysis data for Kaolinite is shown in Figure 7.

## Claims

1. A dosage form of an aerosolisable gel product, wherein the dosage form of the aerosolisable gel comprises
(a) nicotine in an amount of 0.5 to 10 mg;
(b) one or more gel forming materials, wherein the one or more gel forming materials is at least kaolinite;
(c) an aerosol forming material; and
(d) water.

2. The dosage form of an aerosolisable gel product according to claim 1 comprising nicotine in an amount of no greater than 2 wt% based on the total weight of the aerosolisable gel.

3. The dosage form of an aerosolisable gel product according to claim 1 comprising nicotine in an amount of no greater than 1.8 wt% based on the total weight of the aerosolisable gel.

4. The dosage form of an aerosolisable gel product according to any one of claims 1 to 3 wherein kaolinite is present in an amount of 10 to 80 wt.% based on the aerosolisable gel.

5. The dosage form of an aerosolisable gel product according to any one of claims 1 to 4 wherein kaolinite is present in an amount of 20 to 70 wt.% based on the aerosolisable gel.

6. The dosage form of an aerosolisable gel product according to any one of claims 1 to 5 wherein kaolinite is present in an amount of 30 to 60 wt.% based on the aerosolisable gel.

7. The dosage form of an aerosolisable gel product according to any one of claims 1 to 6 wherein the aerosol forming material is selected from glycerol, propylene glycol and mixtures thereof.

8. The dosage form of an aerosolisable gel product according to any one of claims 1 to 7 wherein the aerosol forming material is present in an amount of 10 to 60 wt.% based on the aerosolisable gel.

9. The dosage form of an aerosolisable gel product according to any one of claims 1 to 8 wherein the aerosol forming material is present in an amount of 20 to 40 wt.% based on the aerosolisable gel.

10. The dosage form of an aerosolisable gel product according to any one of claims 1 to 9 wherein water is present in an amount of 1 to 40 wt.% based on the aerosolisable gel.

11. The dosage form of an aerosolisable gel product according to any one of claims 1 to 10 wherein water is present in an amount of 1 to 20 wt.% based on the aerosolisable gel.

12. A method of forming an aerosol, the method comprising the step of heating an aerosolisable gel comprising
(a) an active agent;
(b) a gel forming material, wherein the gel forming material is at least kaolinite ;
(c) an aerosol forming material; and
(d) water.

13. The A method of forming an aerosol according to claim 12 wherein the aerosol forming material is selected from glycerol, propylene glycol and mixtures thereof.

14. An electronic vapour provision system comprising:
(i) an aerosolisable gel comprising
(a) an active agent;
(b) one or more gel forming materials, wherein the one or more gel forming material is at least kaolinite ;
(c) an aerosol forming material; and
(d) water;
(ii) a heater for heating the aerosolisable gel and thereby vaporising some or all of the aerosolisable gel for inhalation by a user of the electronic vapour provision system.

## Patentansprüche

1. Dosierungsform eines aerosolierbaren Gelprodukts, wobei die Dosierungsform des aerosolierbaren Gels umfasst
(a) Nikotin in einer Menge von 0,5 bis 10 mg;
(b) ein oder mehrere gelbildende Materialien, wobei das eine oder die mehreren gelbildenden Materialien mindestens Kaolinit ist;
(c) ein aerosolbildendes Material; und
(d) Wasser.

2. Dosierungsform eines aerosolierbaren Gelprodukts nach Anspruch 1, umfassend Nikotin in einer Menge von nicht mehr als 2 Gew.-%, basierend auf dem Gesamtgewicht des aerosolierbaren Gels.

3. Dosierungsform eines aerosolierbaren Gelprodukts nach Anspruch 1, umfassend Nikotin in einer Menge von nicht mehr als 1,8 Gew.-%, basierend auf dem Gesamtgewicht des aerosolierbaren Gels.

4. Dosierungsform eines aerosolierbaren Gelprodukts nach einem der Ansprüche 1 bis 3, wobei Kaolinit in einer Menge von 10 bis 80 Gew.-%, basierend auf dem aerosolierbaren Gel, vorhanden ist.

5. Dosierungsform eines aerosolierbaren Gelprodukts nach einem der Ansprüche 1 bis 4, wobei Kaolinit in einer Menge von 20 bis 70 Gew.-%, basierend auf dem aerosolierbaren Gel, vorhanden ist.

6. Dosierungsform eines aerosolierbaren Gelprodukts nach einem der Ansprüche 1 bis 5, wobei Kaolinit in einer Menge von 30 bis 60 Gew.-%, basierend auf dem aerosolierbaren Gel, vorhanden ist.

7. Dosierungsform eines aerosolierbaren Gelprodukts nach einem der Ansprüche 1 bis 6, wobei das aerosolbildende Material aus Glycerin, Propylenglykol und Gemischen davon ausgewählt ist.

8. Dosierungsform eines aerosolierbaren Gelprodukts nach einem der Ansprüche 1 bis 7, wobei das aerosolbildende Material in einer Menge von 10 bis 60 Gew.-%, basierend auf dem aerosolierbaren Gel, vorhanden ist.

9. Dosierungsform eines aerosolierbaren Gelprodukts nach einem der Ansprüche 1 bis 8, wobei das aerosolbildende Material in einer Menge von 20 bis 40 Gew.-%, basierend auf dem aerosolierbaren Gel, vorhanden ist.

10. Dosierungsform eines aerosolierbaren Gelprodukts nach einem der Ansprüche 1 bis 9, wobei Wasser in einer Menge von 1 bis 40 Gew.-%, basierend auf dem aerosolierbaren Gel, vorhanden ist.

11. Dosierungsform eines aerosolierbaren Gelprodukts nach einem der Ansprüche 1 bis 10, wobei Wasser in einer Menge von 1 bis 20 Gew.-%, basierend auf dem aerosolierbaren Gel, vorhanden ist.

12. Verfahren zum Bilden eines Aerosols, wobei das Verfahren den Schritt zum Erhitzen eines aerosolierbaren Gels umfasst, umfassend
(a) einen Wirkstoff;
(b) ein gelbildendes Material, wobei das gelbildende Material mindestens Kaolinit ist;
(c) ein aerosolbildendes Material; und
(d) Wasser.

13. Verfahren zum Bilden eines Aerosols nach Anspruch 12, wobei das aerosolbildende Material aus Glycerin, Propylenglykol und Gemischen davon ausgewählt ist.

14. Elektronisches Dampfbereitstellungssystem, umfassend:
(i) aerosolierbares Gel, umfassend
(a) einen Wirkstoff;
(b) ein oder mehrere gelbildende Materialien, wobei das eine oder die mehreren gelbildenden Materialien mindestens Kaolinit ist;
(c) ein aerosolbildendes Material; und
(d) Wasser;
(ii) ein Heizelement zum Erhitzen des aerosolierbaren Gels und dadurch Verdampfen eines Teils oder des gesamten aerosolierbaren Gels zur Inhalation durch einen Benutzer des elektronischen Dampfbereitstellungssystems.

## Revendications

1. Forme de dosage d'un produit de gel pouvant former un aérosol, dans laquelle la forme de dosage du gel pouvant former un aérosol comprend
(a) de la nicotine en une quantité de 0,5 à 10 mg ;
(b) un ou plusieurs matériaux gélifiants, dans laquelle les un ou plusieurs matériaux gélifiants sont au moins de la kaolinite ;
(c) un matériau formant un aérosol ; et
(d) de l'eau.

2. Forme de dosage d'un produit de gel pouvant former un aérosol selon la revendication 1, comprenant de la nicotine en une quantité non supérieure à 2 % en poids sur la base du poids total du gel pouvant former un aérosol.

3. Forme de dosage d'un produit de gel pouvant former un aérosol selon la revendication 1, comprenant de la nicotine en une quantité non supérieure à 1,8 % en poids sur la base du poids total du gel pouvant former un aérosol.

4. Forme de dosage d'un produit de gel pouvant former un aérosol selon l'une quelconque des revendications 1 à 3, dans laquelle la kaolinite est présente en une quantité de 10 à 80 % en poids sur la base du gel pouvant former un aérosol.

5. Forme de dosage d'un produit de gel pouvant former un aérosol selon l'une quelconque des revendications 1 à 4, dans laquelle la kaolinite est présente en une quantité de 20 à 70 % en poids sur la base du gel pouvant former un aérosol.

6. Forme de dosage d'un produit de gel pouvant former un aérosol selon l'une quelconque des revendications 1 à 5, dans laquelle la kaolinite est présente en une quantité de 30 à 60 % en poids sur la base du gel pouvant former un aérosol.

7. Forme de dosage d'un produit de gel pouvant former un aérosol selon l'une quelconque des revendications 1 à 6, dans laquelle le matériau formant un aérosol est choisi parmi le glycérol, le propylène glycol et des mélanges de ceux-ci.

8. Forme de dosage d'un produit de gel pouvant former un aérosol selon l'une quelconque des revendications 1 à 7, dans laquelle le matériau formant un aérosol est présent en une quantité de 10 à 60 % en poids sur la base du gel pouvant former un aérosol.

9. Forme de dosage d'un produit de gel pouvant former un aérosol selon l'une quelconque des revendications 1 à 8, dans laquelle le matériau formant un aérosol est présent en une quantité de 20 à 40 % en poids sur la base du gel pouvant former un aérosol.

10. Forme de dosage d'un produit de gel pouvant former un aérosol selon l'une quelconque des revendications 1 à 9, dans laquelle de l'eau est présente en une quantité de 1 à 40 % en poids sur la base du gel pouvant former un aérosol.

11. Forme de dosage d'un produit de gel pouvant former un aérosol selon l'une quelconque des revendications 1 à 10, dans laquelle de l'eau est présente en une quantité de 1 à 20 % en poids sur la base du gel pouvant former un aérosol.

12. Procédé de formation d'un aérosol, le procédé comprenant l'étape de chauffage d'un gel pouvant former un aérosol comprenant
(a) un agent actif ;
(b) un matériau gélifiant, dans lequel le matériau gélifiant est au moins de la kaolinite ;
(c) un matériau formant un aérosol ; et
(d) de l'eau.

13. Procédé de formation d'un aérosol selon la revendication 12, dans lequel le matériau formant un aérosol est choisi parmi le glycérol, le propylène glycol et des mélanges de ceux-ci.

14. Système de fourniture de vapeur électronique comprenant :
(i) un gel pouvant former un aérosol comprenant
(a) un agent actif ;
(b) un ou plusieurs matériaux gélifiants, dans lequel les un ou plusieurs matériaux gélifiants sont au moins de la kaolinite ;
(c) un matériau formant un aérosol ; et
(d) de l'eau ;
(ii) un élément chauffant pour chauffer le gel pouvant former un aérosol et ainsi vaporiser une partie ou la totalité du gel pouvant former un aérosol pour inhalation par un utilisateur du système de fourniture de vapeur électronique.
